# EUROPEAN PATENT APPLICATION

(11) **EP 3 109 232 A1**
(43) Date of publication of application: **28.12.2016**
(21) Application number: 14882606.8
(22) Date of filing: 16.06.2014
(51) Int. Cl.: C07D 211/42, A61K 31/452, A61P 11/00, A61P 11/08, A61P 29/00

(54) **OPTICALLY ACTIVE FORM OF MEPENZOLATE, AND AMELIORATING AGENT FOR CHRONIC OBSTRUCTIVE PULMONARY DISEASE WHICH CONTAINS SAME AS ACTIVE INGREDIENT**

(30) Priority: 17.02.2014 JP 2014027539
(71) Applicant: LTT Bio-Pharma Co., Ltd., Minato-ku Tokyo 105-0022 (JP)
(72) Inventor: MIZUSHIMA, Toru, Tokyo 105-0022 (JP)
(74) Representative: Beckmann, Claus
(86) International application number: PCT/JP2014/065880
(87) International publication number: WO 2015/122031

(57) **Abstract**

An optically active form of mepenzolate bromide is provided. An agent for ameliorating chronic obstructive pulmonary disease that contains the optically active form as an active ingredient and is based on a bronchodilator effect and an anti-inflammatory effect is also provided. (3R)- or (3S)-3-[(hydroxy)(diphenyl)acetoxy]-1,1-dimethylpiperidinium bromide (hereinafter referred to as "(R)- or (S)-mepenzolate bromide") is provided. An agent for ameliorating chronic obstructive pulmonary disease that contains the (R)- or (S)-mepenzolate bromide as an active ingredient is provided. Furthermore, an agent for ameliorating chronic obstructive pulmonary disease whose mode of administration is airway administration or inhalation administration is provided.

## Description

### TECHNICAL FIELD

The present invention relates to an agent for ameliorating chronic obstructive pulmonary disease. Specifically, the present invention relates to an agent for ameliorating chronic obstructive pulmonary disease, containing an optically active form of mepenzolate bromide (hereinafter may also be referred to simply as "mepenzolate") as an active ingredient.

### BACKGROUND ART

Chronic obstructive pulmonary disease (COPD: chronic obstructive pulmonary disease, hereinafter may be referred to as "COPD") is a disease in which various factors, particularly smoking, causes chronic lung inflammation and the inflammation causes, for example, alveolar destruction and bronchial mucous gland hypertrophy, which in turn leads to shortness of breath, increased coughing or expectoration, and the like.

A disease previously referred to as pulmonary emphysema (PE) and a disease previously referred to as chronic bronchitis (CB) are often combined in various ratios and developed, and accordingly, the diseases caused by these two diseases are currently collectively referred to as chronic obstructive pulmonary disease (COPD).

According to a trial calculation made by the World Health Organization (WHO), three million people died of COPD worldwide in one year in 2005 and COPD is the fourth leading cause of death, and it is predicted that the number of deaths from COPD will increase further by 30% in the next 10 years. According to statistics by the Ministry of Health, Labor and Welfare in Japan, in 2005, deaths from COPD accounted for 1.3% of the total number of Japanese deaths, and COPD is the tenth leading cause of death and the seventh leading cause of death exclusively in men.

The primary cause of COPD development is smoking. 90% of COPD patients are smokers (Non-Patent Literature 1) and the risk of smokers developing COPD is six or more times higher than that of nonsmokers. Approximately 10 to 15% of smokers develop COPD and, exclusively in the elderly population, nearly 50% of smokers suffer from COPD. Other causes include indoor air pollution or air pollution, inhalation of chemical substances or dust, genetic factors, pneumonia or bronchitis in childhood, and the like.

COPD is a disease whose characteristic condition is airflow limitation, that is, difficulty in breathing out, although the true nature of the condition is chronic airway inflammation. Smoking, inhaled substances, and the like cause inflammation in various sites in a lung ranging from a central airway to a peripheral bronchus. It is believed that the inflammation leads to protease-antiprotease imbalance, oxidant-antioxidant imbalance, and the like, which in turn causes, for example, alveolar destruction and bronchial mucous gland hypertrophy.

COPD is an incurable disease since irreversible destruction of the airway has occurred. Smoking cessation, pharmacotherapy by administration of, e.g., a bronchodilator, an expectorant, and an antitussive drug, oxygen therapy, or the like can only relieve symptoms of COPD, and thus COPD is a very troublesome disease.

From the above-mentioned point of view, various kinds of agents for ameliorating COPD or methods for ameliorating COPD have been proposed so far (for example, Patent Literatures 1 and 2), however, development of an even better agent for ameliorating COPD is currently awaited.

In the above-mentioned context, the present inventors have performed a study that searches existing commercially available drugs to develop an agent for ameliorating COPD. As a result, the present inventors have confirmed that mepenzolate bromide (brand name: Trancolon (registered trademark)), which has been clinically used as a therapeutic drug for irritable bowel, exhibits a therapeutic effect on COPD based on a bronchodilator effect and an anti-inflammatory effect thereof, and have filed a patent application (Patent Literature 3).

In this specification, the present inventors carried out further research and focused on the chemical structure of mepenzolate bromide. The present inventors have found that both an R-form of mepenzolate bromide and an S-form of mepenzolate bromide that were obtained by producing mepenzolate bromide as two optically active forms have an excellent effect of ameliorating COPD and thus have accomplished the present invention.

Mepenzolate bromide is known as an anticholinergic drug that has an effect of suppressing movement and contraction in a lower gastrointestinal tract and has been used as a therapeutic drug for irritable bowel in a clinical setting since 1967. However, it is unknown that mepenzolate bromide is effective for COPD treatment. It has also been unknown so far that individual optically active forms (namely, an R-form and an S-form) of mepenzolate bromide that were produced as optically active forms also have both a bronchodilator effect and an anti-inflammatory effect and thus have an effect of improving COPD treatment.

### PRIOR ART DOCUMENTS

### PATENT LITERATURE

Patent Literature 1: Japanese Patent Application Laid-Open No. 2006-56890
Patent Literature 2: Japanese Patent Application Laid-Open No. 2008-189667
Patent Literature 3: WO 2013/137009

### NON-PATENT LITERATURE

Non-Patent Literature 1: Annual Review of Medicine, Vol. 40, p 411-429 (1989)

### SUMMARY OF THE INVENTION

### PROBLEMS TO BE SOLVED BY THE INVENTION

Therefore, in view of the above-mentioned present circumstances, it is an object of the present invention to provide an optically active form of mepenzolate bromide and to provide an agent for ameliorating chronic obstructive pulmonary disease that contains such an optically active form as an active ingredient.

### MEANS FOR SOLVING THE PROBLEM

To achieve the above-mentioned object, in a basic aspect, the present invention provides (3R)-3-[(hydroxy)(diphenyl)acetoxy]-1,1-dimethylpiperidinium bromide (hereinafter referred to as "(R)-mepenzolate bromide") that has a configuration represented by the following formula:

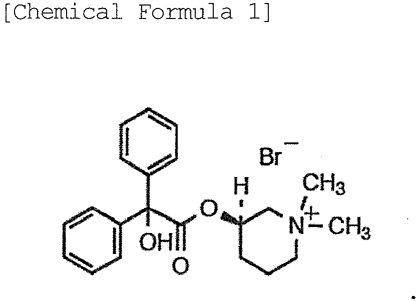

In another basic aspect, the present invention provides (3S)-3-[(hydroxy)(diphenyl)acetoxy]-1,1-dimethylpiperidinium bromide (hereinafter referred to as "(S)-mepenzolate bromide") that has a configuration represented by the following formula:

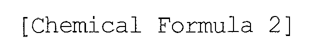

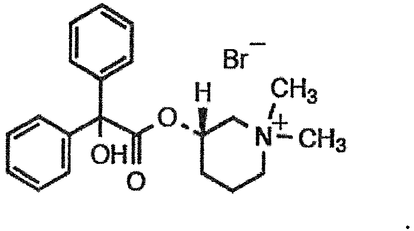

Furthermore, in still another aspect, the present invention provides an agent for ameliorating chronic obstructive pulmonary disease characterized in that it contains (R)- or (S)-mepenzolate bromide represented by each of the above-mentioned formulae as an active ingredient. More specifically, the present invention provides an agent for ameliorating chronic obstructive pulmonary disease whose mode of administration is airway administration or inhalation administration.

Furthermore, the present invention provides a method for producing (R)- or (S)-mepenzolate bromide represented by each of the above-mentioned formulae. Specifically, the present invention provides a method for producing (R)- or (S)-mepenzolate bromide including allowing benzilic acid to react with (R)- or (S)-3-hydroxy-1-methylpiperidin in the presence of a condensing agent and then allowing the obtained product to be treated with methyl bromide.

### EFFECTS OF THE INVENTION

The present invention provides an agent for ameliorating COPD that contains (R)- or (S)-mepenzolate bromide, which is an optically active form of mepenzolate bromide, as an active ingredient, wherein mepenzolate bromide has been already used as a therapeutic drug for irritable bowel and the safety thereof has been confirmed.

(R)- or (S)-mepenzolate bromide, which is an active ingredient of the agent for ameliorating COPD provided by the present invention, is an optically active form of clinically used mepenzolate bromide. (R)- or (S)-mepenzolate bromide has an excellent effect of ameliorating COPD and in particular exhibits a significant effect by airway administration and inhalation administration.

The effect of (R)- or (S)-mepenzolate bromide is based on a bronchodilator effect and an anti-inflammatory effect. Since there has been, until now, no agent for ameliorating COPD that has both of these effects, the agent for ameliorating COPD of the present invention is a very specific agent for ameliorating COPD.

Furthermore, a feature of (R)- or (S)-mepenzolate bromide, which is an active ingredient of the agent for ameliorating COPD provided by the present invention, is that it shows the effects thereof by an action mechanism different from those of a muscarinic antagonistic action and an anticholinergic action.

Therefore, in a situation where there has been, until now, no effective agent for ameliorating COPD, the specific and safe (R)- or (S)-mepenzolate bromide can be administered to ameliorate the symptoms of COPD, and thus, the medical effect thereof is very specific.

### BRIEF DESCRIPTION OF DRAWINGS

Fig. 1 is a graph showing the results of the receptor binding ability (in vitro) assay in Test Example 2.1, specifically, the results of binding in the presence of [³H]NMS.
Fig. 2 is a graph expressing the receptor binding ability (in vitro) measured in Test Example 2.1 as an IC₅₀ value.
Fig. 3 is a graph showing the results of the receptor (M3 receptor) binding ability assay in Test Example 2.2.
Fig. 4 is a graph showing the results of the receptor (M2 receptor) binding ability assay in Test Example 2.2.
Fig. 5 is a graph showing the results of measurement of airway resistance after administration of mepenzolate bromide (a racemate, an R-form, and an S-form) at a dose of 3.75 µg/kg in Test Example 3.1.
Fig. 6 is a graph showing the results of measurement of airway resistance after administration of mepenzolate bromide (a racemate, an R-form, and an S-form) at a dose of 38 µg/kg in Test Example 3.2.
Fig. 7 is a graph showing the results of measurement of airway resistance after administration of mepenzolate bromide (a racemate, an R-form, and an S-form) at a dose of 3.8 µg/kg in Test Example 3.2.
Fig. 8 shows graphs showing the results of measurement of a total cell count and a neutrophil count in Test Example 4.1.
Fig. 9 shows a graph showing the results of measurement of a total cell count in Test Example 4.2.
Fig. 10 shows a graph showing the results of measurement of a neutrophil count in Test Example 4.2.
Fig. 11 is a graph showing the results of measurement of TNF-α in Test Example 4.3.
Fig. 12 is a graph showing the results of measurement of MIP-2 in Test Example 4.3.
Fig. 13 is a graph showing the results of measurement of NCP-1 in Test Example 4.3.
Fig. 14 is a graph showing the results of measurement of KC in Test Example 4.3.
Fig. 15 is a graph showing the results of measurement of the fecal pellet output in Test Example 5.
Fig. 16 is a graph showing the results of measurement of the heart rate in Test Example 6.

### EMBODIMENTS FOR CARRYING OUT THE INVENTION

(R)- or (S)-mepenzolate bromide used in the agent for ameliorating COPD provided by the present invention is an optically active form of clinically used mepenzolate bromide.

This clinically used mepenzolate bromide is listed as a substance having the following structural formula in the Japanese Pharmacopoeia 16th Edition.

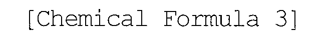

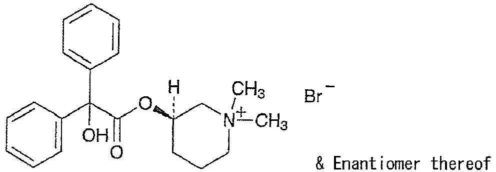

Specifically, mepenzolate bromide is shown by writing a conformational formula of the structure of one optically active form and adding a description "and an enantiomer thereof" in accordance with the rule for describing a racemate (a mixture of equal quantities of enantiomers).

Specifically, (R)- or (S)-mepenzolate bromide of the present invention can be produced by a production method described below.

Thus, when a method for producing (R)-mepenzolate bromide is illustrated, (R)-mepenzolate bromide can be produced by following a chemical formula described below.

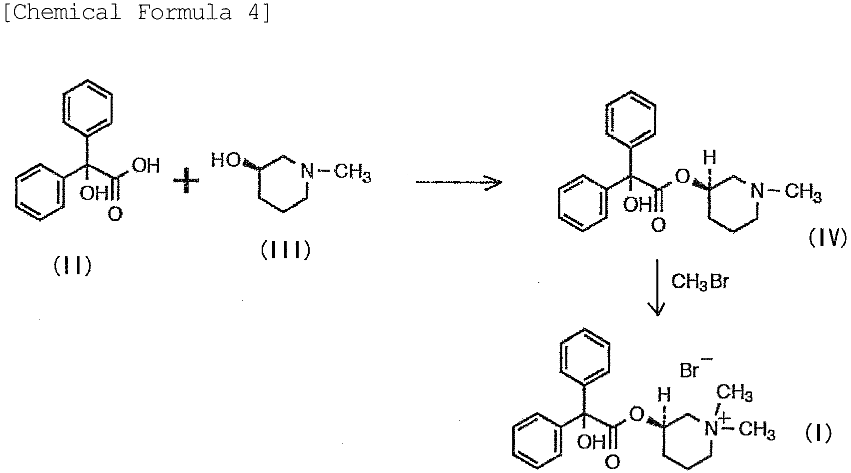

Briefly, (R)-mepenzolate bromide (I) is produced by allowing benzilic acid (II) to react with (R)-3-hydroxy-1-methylpiperidin (III) in a suitable organic solvent in the presence of a condensing agent and then allowing the resulting product (IV) (hereinafter described as "(R)-t-mepenzolate") to be treated with methyl bromide.

The organic solvent to be used is not particularly limited as long as it does not affect the reaction. However, preferably, a solvent such as dimethylformamide (DMF) or diethylformamide (DEF) is favorable.

Preferably, CDI (carbonyldiimidazole) may be used as a condensing agent used in the reaction.

The reaction is performed by dissolving both the compound of the formula (II) and the compound of the formula (III) as well as CDI as a condensing agent in, for example, DMF, and stirring the mixture at the temperature of approximately 30 to 100°C for 1 to 24 hours. After completion of the reaction, a routine after-treatment known per se (extraction, solvent removal by evaporation, chromatography, and the like) can be performed to obtain objective (R)-t-mepenzolate represented by the formula (IV).

The obtained (R)-t-mepenzolate (IV) is used without purification and stirred with methyl bromide at room temperature or under moderate heating in an organic solvent such as an alcohol or acetonitrile. In this manner, (R)-mepenzolate bromide represented by the formula (I), which is the target compound of the present invention, can be obtained as a crystal.

The above description is about a method for producing (R)-mepenzolate bromide, however, when (S)-mepenzolate bromide is to be obtained, objective (S)-mepenzolate bromide can be obtained in the same method, wherein (S)-3-hydroxy-1-methylpiperidin is used instead of (R)-3-hydroxy-1-methylpiperidin of the formula (III) in the above-mentioned chemical reaction formula.

The agent for ameliorating COPD provided by the present invention contains (R)- or (S)-mepenzolate bromide represented by the chemical formula having the above-mentioned configuration as an active ingredient and can be administered preferably by airway administration and inhalation administration.

An airway-administered formulation for the above-mentioned airway administration and an inhalation formulation for the above-mentioned inhalation administration represent a pharmaceutical composition to be delivered to respective tissues such as a trachea, a bronchus, and a lung, and favorably represent a nasal drop or a composition suitable for transnasal administration or pulmonary administration. These formulations are effective when administered by a nebulizer, an atomizer, a dropper, a pipette, a cannula, and the like.

In such a case, the airway-administered formulation and the inhalation formulation can be prepared in the form of a powder formulation, a solution formulation, or a suspension formulation of (R)- or (S)-mepenzolate bromide.

When the airway-administered formulation and the inhalation formulation are prepared as a powder formulation, the formulations can be prepared by processing (R)- or (S)-mepenzolate bromide as an active ingredient into fine particles alone or together with a suitable additive such as an excipient, a lubricant, a binder, a disintegrant, a stabilizer, or a flavoring agent.

Furthermore, when the airway-administered formulation and the inhalation formulation are prepared as a solution formulation or a suspension formulation, the formulations can be prepared as follows. For example, the formulations can be prepared by dissolving or suspending (R)- or (S)-mepenzolate bromide in water or a mixture that is a mixed solvent of water and a cosolvent, for example, an alcoholic cosolvent such as ethanol, propylene glycol, or polyethylene glycol.

Such solution or suspension may further contain an antiseptic, a solubilizer, a buffering agent, an isotonic agent, an absorption promoter, a thickener, and the like.

The airway-administered formulation and the inhalation formulation prepared as described above are administered to a nasal cavity or an oral cavity or directly to a tissue such as a trachea, a bronchus, or a lung by means common in the field of an inhalation formulation. For example, these formulations are administered by using a dropper, a pipette, a cannula, or a sprayer such as an atomizer or a nebulizer to pulverize the formulations.

When a sprayer is used, the formulations can be sprayed as an aerosol prepared in the form of a pressurized package together with a suitable propellant (for example, a chlorofluorocarbon such as dichlorofluoromethane, trichlorofluoromethane, or dichlorotetra-fluoromethane, or a gas such as carbon dioxide) or can be administered by using a nebulizer.

The dosage of (R)- or (S)-mepenzolate bromide that is an active ingredient of the agent for ameliorating COPD of the present invention varies depending on a method by which the formulation is prepared, a dosage form, a symptom of the disease, the age and weight of a patient, and the like and thus cannot be necessarily specified. However, by way of example, a clinical dosage is 0.5 to 200 mg per day for adults in the case of administration of an airway-administered formulation and an inhalation formulation. The frequency of administration cannot be necessarily specified, either, however, the frequency of administration can be once or several times a day.

Furthermore, (R)- or (S)-mepenzolate bromide that is an active ingredient of the agent for ameliorating COPD of the present invention can be used more effectively by combined administration with an anticholinergic drug such as ipratropium, scopolamine, pirenzepine, tiotropium, or oxitropium.

### EXAMPLES

Hereinbelow, the present invention will be described in more detail with reference to specific Test Examples and Working Examples, but the present invention is not limited to these descriptions.

"Mep" or "(±)-Mep" described in the figures refers to a racemate of mepenzolate bromide; "R-Mep" or "(R)-Mep" refers to (R)-mepenzolate bromide (an R-form); and "S-Mep" or "(S)-Mep" refers to (S)-mepenzolate bromide (an S-form). Furthermore, "PPE" refers to porcine pancreatic elastase.

* in the figures indicates p < 0.05 and ** indicates p < 0.01.

In the following Examples, various instrumental analyses were performed by the following measurement methods.

### <Measurement Method>

IR: Jeol FT-IR SPX60 spectrometer, thin film method
¹H NMR: Agilent 400-MR spectrometer, in DMSO, 400 MHz
¹³C NMR: Agilent 400-MR spectrometer, in DMSO, 100 MHz
Highly analytical mass spectrum (HRMS): Jeol JMS-700 MStation
Optical rotation: Jasco P-1010 polarimeter

### Example 1: Synthesis of (R)-Mepenzolate Bromide (an R-form)

830 mg (3.6 mmol) of benzilic acid was added to a solution of 883 mg (5.4 mmol) of carbonyldiimidazole (CDI) dissolved in 8 mL of DMF and was stirred at room temperature for 15 minutes. A solution of 500 µL (4.3 mmol) of (R)-3-hydroxy-1-methylpiperidin dissolved in 4 mL of DMF was dropwisely added into this solution at 80°C. Subsequently, the mixture was stirred at 80°C for 18 hours. The reaction was stopped by adding water at room temperature. An organic compound was extracted with ethyl acetate. The extract was collected, washed with saline, and dried over sodium sulfate, and the solvent was concentrated under reduced pressure. The resulting residue was subjected to short-column chromatography on silica gel and eluted with ethyl acetate, and then, the eluate was redissolved in dichloromethane. The obtained solution was washed with an aqueous solution of sodium hydrogencarbonate and saline, dried over sodium sulfate, and the solution was concentrated in vacuo to yield 860 mg (72%) of (R)-t-mepenzolate as a yellow oily substance.

This product was used in the next step without purification.

500 mg (1.5 mmol) of the (R)-t-mepenzolate obtained above was dissolved in 6 mL of acetonitrile and 3.6 mL (7.0 mmol) of methyl bromide was added to this solution. The mixture was stirred at room temperature for 5 hours. A precipitate was recovered by filtration and washed with ethyl ether to yield 570 mg (77%) of (R)-mepenzolate bromide (an R-form) as a colorless solid.

IRᵥₘₐₓ: 3432, 3315, 1735, 1216, 1093, 1068, 923, 705 cm⁻¹
¹HNMR (CDCl₃) δ: 7.28 - 7.37 (m, 10H), 6.82 (s, 1H), 5.28 (m, 1H), 3.63 (dd, J = 13.5, 3.5 Hz, 1H), 3.49 (dd, J = 13.5, 4.7 Hz, 1H), 3.37 (m, 1H), 3.33 (m, 1H), 3.10 (s, 3H), 2.82 (s, 3H), 1.63 - 1.85 (m, 4H)
¹³CNMR (CDCl₃) δ: 171.9, 143.0, 142.7, 128.0, 127.9, 127.7, 127.6, 127.0, 80.7, 66.8, 61.6, 60.8, 25.2, 16.2,
[α]_{D}²⁵: -8.12 (c 1.00, MeOH)
Melting Point: 224.2 to 224.9°C
HRMS (FAB): calculated for C₁₂H₁₉O₅: [M+1]⁺: 340.1913, a found value: m/z = 340.1905

### Example 2: Synthesis of (S)-Mepenzolate Bromide (an S-form)

(S)-mepenzolate bromide was obtained as a colorless solid by the same method as that described in Working Example 1, except that (S)-3-hydroxy-1-methylpiperidin was used instead of (R)-3-hydroxy-1-methylpiperidin. The yield was 72%.

IRᵥₘₐₓ: 3436, 3299, 1733, 1214, 1097, 1066, 929 cm⁻¹
¹H NMR (CDCl₃) δ: 7.28 - 7.37 (m, 10H), 6.81 (s, 1H), 5.27 (m, 1H), 3.65 (dd, J = 13.4, 3.4 Hz, 1H), 3.51 (dd, J = 13.4, 4.6 Hz, 1H), 3.36 (m, 1H), 3.32 (m, 1H), 3.09 (s, 3H), 2.81 (s, 3H), 1.63 - 1.85 (m, 4H)
¹³C NMR (CDCl₃) δ: 171.9, 143.0, 142.7, 128.0, 127.9, 127.7, 127.6, 127.0, 80.7, 66.8, 61.6, 60.8, 25.2, 16.2
[α]_{D}²⁵: +8.33 (c 1.00, MeOH)
Melting Point: 223.1 to 223.8°C
HRMS (FAB): calculated for C₁₂H₁₉O₅: [M+1]⁺: 340.1913, a found value: m/z = 340.1928

### Test Example 1: Measurement of the Concentration in a Lung Tissue of Airway-Administered Mepenzolate Bromide (an R-form and an S-form)

### <Method>

An R-form of mepenzolate bromide or an S-form of mepenzolate bromide was administered to 4 to 6 week old ICR mice via the airway at a dose of 20 mg/kg. The amount of each mepenzolate bromide in the lung tissue (the concentration in the lung tissue) was measured 5, 15, and 30 minutes after administration.

The whole lungs of the mice were excised at each of the measurement times after administration of mepenzolate bromide and homogenized in phosphate buffered saline (PBS) containing 50 U/mL heparin, and centrifuged to obtain test samples. The test samples were divided into 300 µL aliquots and subjected to ultrafiltration using an Amicon ultra-0.5 centrifugal filter. The obtained samples were subjected to high-performance liquid chromatography (HPLC) using Daicel Chiralcel 1A (0.46 x 25 cm) to determine the amount of mepenzolate bromide in the cellular tissues.

The HPLC conditions were as follows.
Detector: Waters 2695 Alliance separation module and Waters 2996 photodiode array detector (manufactured by Waters Corporation)
Eluent: Solvent A (Water containing 0.1 M hexafluorophosphate) / Solvent B (acetonitrile)
Flow Rate: 1.0 mL/min
Mobile Phase: 70% Solvent A / 30% Solvent B
Measuring wavelength: 220 nm

### <Results>

The results are shown in Table 1 below.

**[Table 1]**

| Comp. | Concentration in lung tissue (µg/lung) | | | | | |
|---|---|---|---|---|---|---|
| | (R)-Mep | | | (S) -Mep | | |
| | 5 min. | 15 min. | 30 min. | 5 min. | 15 min. | 30 min. |
| R-form | 44.5±11.5 | 6.3±4.2 | 5.1±1.3 | n.d. | n.d. | n.d. |
| S-form | n.d. | n.d. | n.d. | 36.4±9.5 | 7.6±0.5 | 6.2±0.4 |

n.d.: not detected

As is found from the results in the table, the R-form and the S-form of mepenzolate bromide administered via the airway were confirmed to remain in the lung tissue while maintaining the optical activity thereof.

Furthermore, on the basis of the fact that the concentration in the tissue decreased over time after administration, it was understood that the drug administrated via the airway had been transferred throughout the body.

### Test Example 2.1: Receptor Binding Ability (In Vitro) (1)

### <Method>

A membrane fraction of CHO-K1 cells overexpressing a human M3 muscarinic receptor was used to measure receptor binding ability by competitive inhibition assay with 1-[N-methyl-³H]scopolamine methyl bromide ([³H]NMS, specific activity: 82 Cimmol). The protein concentration of the cell membrane was 5.0 µg/well and the [³H]NMS concentration was 2.0 nM.

The concentration range of the inhibitor (mepenzolate) (10⁻¹⁴ to 10⁻⁵ M) was tested by generating a comparison curve. Nonspecific binding was measured in the presence of 1 µM atropine.

A binding buffer (phosphate buffer/saline) was added to the reagents to make a total volume of 200 µL. The mixed reaction solution was incubated at room temperature for 2 hours so as to reach equilibrium, and then, filtered with a GF/C filter plate that had been pretreated with a washing buffer containing 1.0% polyethyleneimine for 1 hour.

Bound [³H]NMS and free [³H]NMS were separated by rapid vacuum filtration and washed three times with a rinse buffer (50 mM Tris, 100 mM saline, pH 7.4). The resulting filter, which was dried and a solid scintillator, MeltiLex A (manufactured by PerkinElmer, Inc.) was melted thereon, was measured and quantified by using a β-counter (MicroBeta microplate scintillation counter (Trilux: manufactured by PerkinElmer, Inc.).

### <Results>

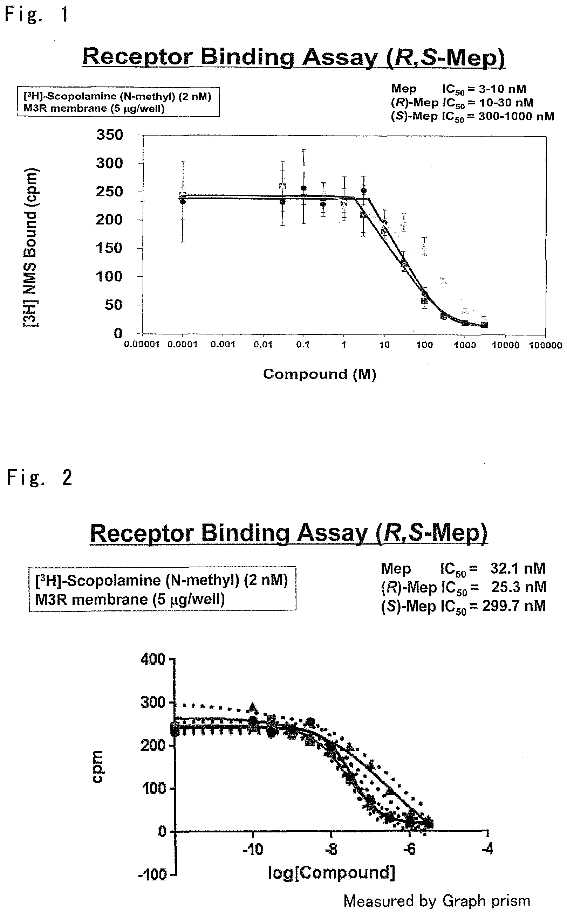

The results were shown in Fig. 1.

As is found from the results shown in Fig. 1, the racemate of mepenzolate bromide (●) and the R-form of mepenzolate bromide (■) bound strongly to the M3 receptor to a similar extent, while the S-form (▼) had a weaker binding ability.

Furthermore, the results representing their receptor binding ability by the 50% inhibition concentrations (IC₅₀) thereof were shown in Fig. 2.

As is also found from the results shown in the figure, the R-form of mepenzolate bromide (■) (IC₅₀ = 25.3 nM) had a tendency to have a stronger binding ability to the M3 receptor than the racemate of mepenzolate bromide (●) (IC₅₀ = 32.1 nM), and the S-form (▼) had a weaker binding ability (IC₅₀ = 299.7 nM).

### Test Example 2.2: Receptor Binding Ability (In Vitro) (2)

### <Method>

Membrane fractions of CHO-K1 cells overexpressing a human M3 muscarinic receptor (hM₃R) or CHO-K1 cells overexpressing a human M2 muscarinic receptor (hM₂R) were used to measure receptor binding ability by competitive inhibition assay with 1-[N-methyl-³H]scopolamine methyl bromide ([³H]NMS, specific activity: 85.5 Cimmol). The protein concentration of the cell membrane was 10.0 µg/well and the [³H]NMS concentration was 2.0 nM.

The concentration range of the inhibitor (mepenzolate) (10⁻¹⁰ to 3 x 10⁻⁵ M) was tested by generating a comparison curve. Nonspecific binding was measured in the presence of 2.5 µM atropine.

A binding buffer (phosphate buffer/saline) was added to the reagents to make a total volume of 200 µL. The mixed reaction solution was incubated at room temperature for 2 hours so as to reach equilibrium, then, filtered with a GF/C filter plate that had been pretreated with a rinse buffer (50 mM Tris, 100 mM saline, pH 7.4) containing 1.0% polyethyleneimine for 1 hour, and washed with an ice-cold rinse buffer four times.

The resulting filter was dried for 30 minutes, and then, fixed to a solid scintillator, MeltiLex A (melt-on scintillation sheet; manufactured by PerkinElmer, Inc.). The radioactivity remaining on the filter was measured in a MicroBeta Trilux microplate scintillation counter (manufactured by PerkinElmer, Inc.).

An experimental IC₅₀ was calculated by using an experimentally obtained constant (κd) of [³H]NMS for hM₃R or hM₂R and the concentration thereof, and then, the affinity at equilibrium was determined as a competitive equilibrium dissociation constant (κi) value. In this regard, the κi value was calculated from three individual curves and modified by using Prism (produced by GraphPad Software, Inc.).

### <Results>

The κi values of the racemate of mepenzolate bromide, (R)-mepenzolate bromide, and (S)-mepenzolate bromide for hM₂R or hM₃R are shown in Table 2 below.

**[Table 2]**

| Test Compound | κi (nM) | |
|---|---|---|
| | hM₂R | hM₃R |
| (R) -Mep | 0.45±0.13 | 2.11±0.23 |
| (S)-Mep | 2.52±0.64 | 28.0±1.70 |
| (±)-Mep | 0.68±0.01 | 2.60±0.22 |

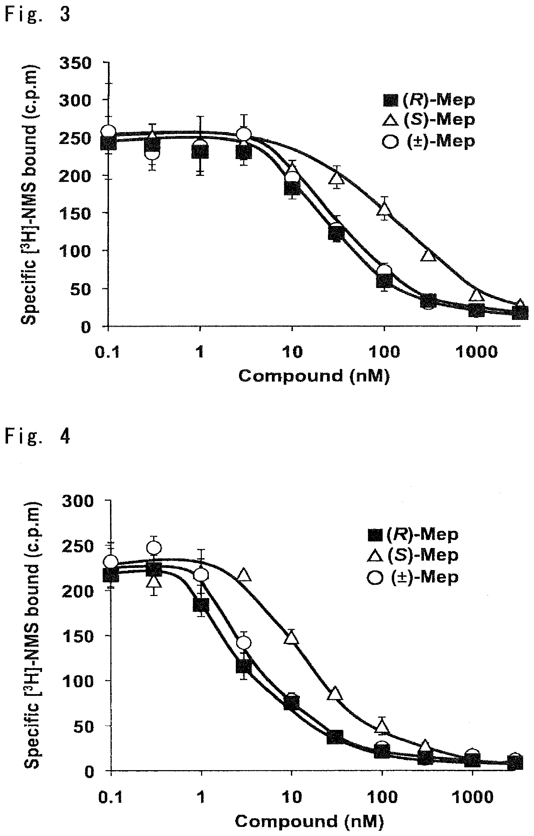

Furthermore, changes in binding abilities at various concentrations were examined and the results were shown in Fig. 3 and Fig. 4.

Fig. 3 shows the results for hM₃R (M3 receptor) and Fig. 4 shows the results for hM₂R (M2 receptor).

From the results shown in Table 2 and the results shown in Fig. 3, it was found that (S)-mepenzolate bromide (Δ) has a lower affinity to hM₃R (M3 receptor) compared to (R)-mepenzolate bromide (■) and the racemate of mepenzolate bromide (○).

### Test Example 3.1: Effect on Methacholine-Induced Airway Constriction (1)

### <Method>

Increase in airway resistance induced by methacoline was measured.

4 to 6 week old ICR mice received 1 mg/mL methacoline by nebulization over 20 seconds and this was repeated five times. After each administration of methacoline was completed, the airway resistance was measured by a snap shot technique. All the data were analyzed by using FlexiVent software.

A racemate, an R-form, or an S-form of mepenzolate bromide each at 3.75 µg/kg was administered to the mice via the airway. One hour after administration, the mice received five exposures to nebulized methacoline by the above-mentioned method and the airway resistance at the time of each administration was measured.

### <Results>

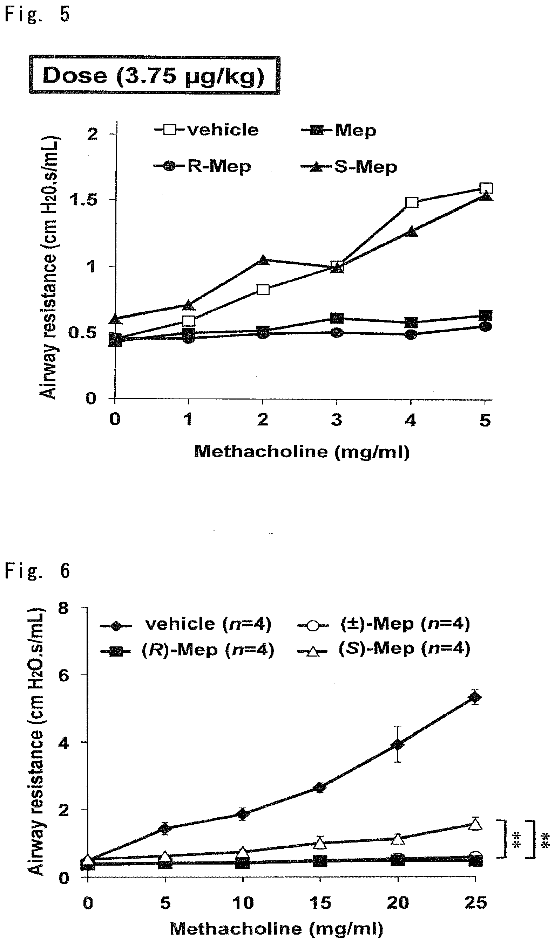

The results were shown in Fig. 5.

As is found from the results shown in Fig. 5, the S-form of mepenzolate bromide (▼) showed little effect, while the racemate (■) and the R-form (●) showed a significant effect of suppressing airway resistance, and the R-form was slightly superior.

### Test Example 3.2: Effect on Methacholine-Induced Airway Constriction (2)

### <Method>

Increase in airway resistance induced by methacoline was measured.

4 to 6 week old ICR mice were anesthetized with chloral hydrate (500 mg/kg), a tracheostomy was performed, and a metal tube 8 mm in diameter was inserted into the trachea.

The mice were mechanically ventilated at 150 respirations/minute with a displacement per one cycle of 8.7 mL/kg and a positive end-expiratory pressure of 2 to 3 cmH₂O.

The mice were exposed to nebulized methacholine (5 mg/kg) for 20 seconds four times at intervals of 40 seconds to induce airway resistance by methacholine, and the airway resistance was measured by a snap shot technique.

A racemate, an R-form, or an S-form of mepenzolate bromide each at 38 µg/kg and 3.8 µg/kg was administered to the mice via the airway. One hour after administration, the mice received five exposures to nebulized methacoline by the above-mentioned method and the airway resistance at the time of each administration was measured.

The data were analyzed by using FlexiVent software.

### <Results>

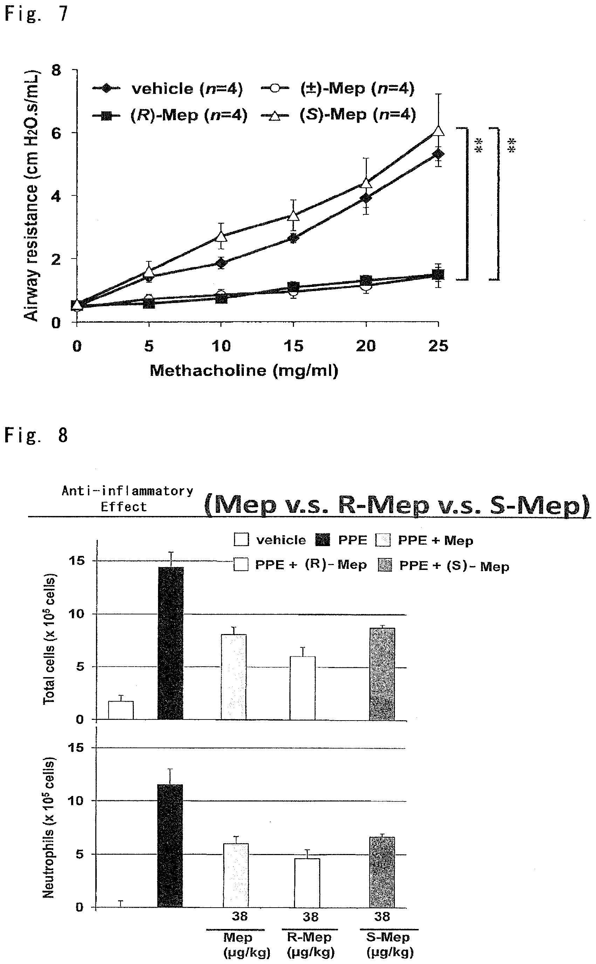

The results were shown in Fig. 6 (a dose of 38 µg/kg) and Fig. 7 (a dose of 3.8 µg/kg).

In the case of a low dose (Fig. 7, a dose of 3.8 µg/kg), the S-form (Δ) of mepenzolate bromide showed little effect, while the racemate (○) and the R-form (■) showed a significant effect of suppressing airway resistance. In the case of a high dose (Fig. 6, a dose of 38 µg/kg), the S-form (Δ) of mepenzolate bromide was found to show a similar effect of suppressing airway resistance to the racemate (○) and the R-form (■).

### Test Example 4.1: Effect on Porcine Pancreatic Elastase (PPE)-Induced Inflammation (1)

### <Method>

Each of a racemate, an R-form, and an S-form of mepenzolate bromide was administered to 4 to 6 week old ICR mice via the airway once at a dose of 38 µg/kg.

100 µg of porcine pancreatic elastase (PPE) per mouse was administered one hour after administration of mepenzolate bromide. Then, broncho alveolar lavage fluid (BALF) was collected from the lung 24 hours after administration of porcine pancreatic elastase and a total cell count and a neutrophil count were measured.

### <Results>

The results were shown in Fig. 8.

As is found from the results shown in Fig. 8, the R-form of mepenzolate bromide had a relatively stronger effect of decreasing both of the total cell count and the neutrophil count compared to the racemate and the S-form, which were similarly effective.

### Test Example 4.2: Effect on Porcine Pancreatic Elastase (PPE)-Induced Inflammation (2)

### <Method>

Each of a racemate, an R-form, and an S-form of mepenzolate bromide was administered to 4 to 6 week old ICR mice via the airway once at a dose of 7.5 µg/kg and 38 µg/kg and an investigation for comparison of administration doses was performed.

20 U/kg of porcine pancreatic elastase (PPE) was administered one hour after administration of mepenzolate bromide. Then, broncho alveolar lavage fluid (BALF) was collected from the lung 6 hours after administration of porcine pancreatic elastase. The BALF was collected by washing the lung twice with saline containing heparin (50 U/mL) with the aid of a tracheostomy tube.

A total cell count of the collected BALF was measured by hemocytometer. The cells were centrifuged by using Cytospin 4 (registered trademark, manufactured by Thermo Electron Corporation) and then fixed with Diff-Quik reagent, and the neutrophil count was determined on the basis of the ratio of the neutrophil count to the total cell count.

### <Results>

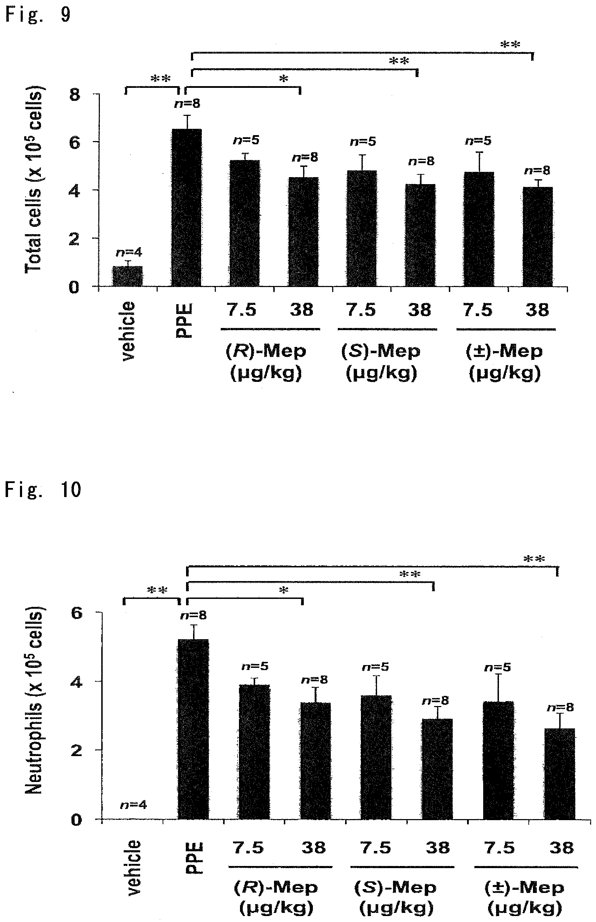

The results were shown in Fig. 9 (total cell count) and Fig. 10 (neutrophil count).

As is found from the results shown in the figures, administration of mepenzolate bromide produced an effect of decreasing both the total cell count and the neutrophil count and there was no significant difference in the decreasing effect among the racemate, the R-form, and the S-form of mepenzolate bromide.

Furthermore, there was no significant difference in the effect among different administration doses.

### Test Example 4.3: Effect on Porcine Pancreatic Elastase (PPE)-Induced Inflammation (3)

The levels of cytokine and chemokine in broncho alveolar lavage fluid (BALF) were measured as indications of an effect on PPE-induced inflammation.

Specifically, TNF-α (tumor factor-α), which is a pro-inflammatory cytokine, was measured as a cytokine that is an inflammatory factor and a macrophage inflammatory protein (MIP-2), a monocyte chemotactic protein (MCP-1), and a keratinocyte-derived chemokine (KC) were measured as an inflammatory chemokine.

### <Method>

This Test Example was performed in the same method as that described in the above "Test Example 4.2" and each of a racemate, an R-form, and an S-form of mepenzolate bromide was examined at a dose of 38 µg/kg.

The measurement of such a cytokine and chemokines in the BALF was performed by ELISA.

### <Results>

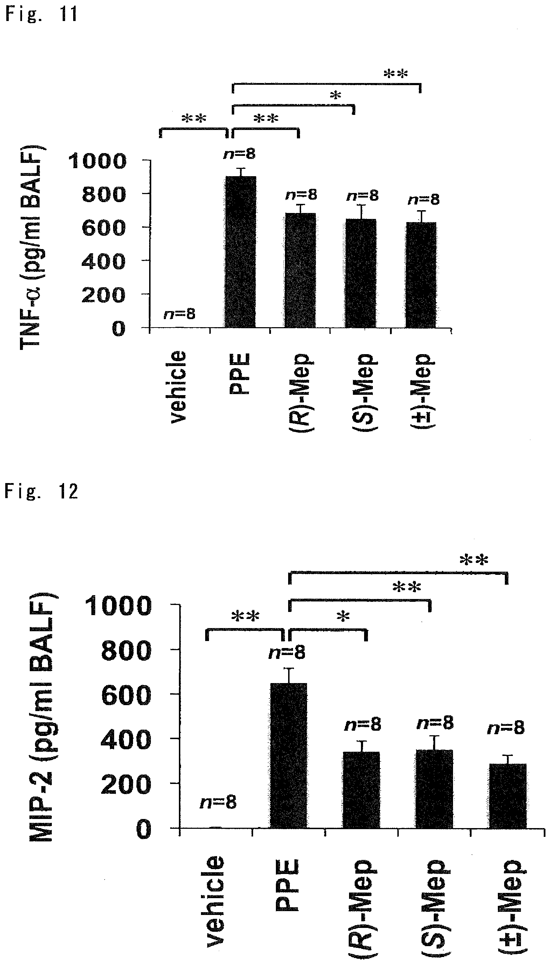

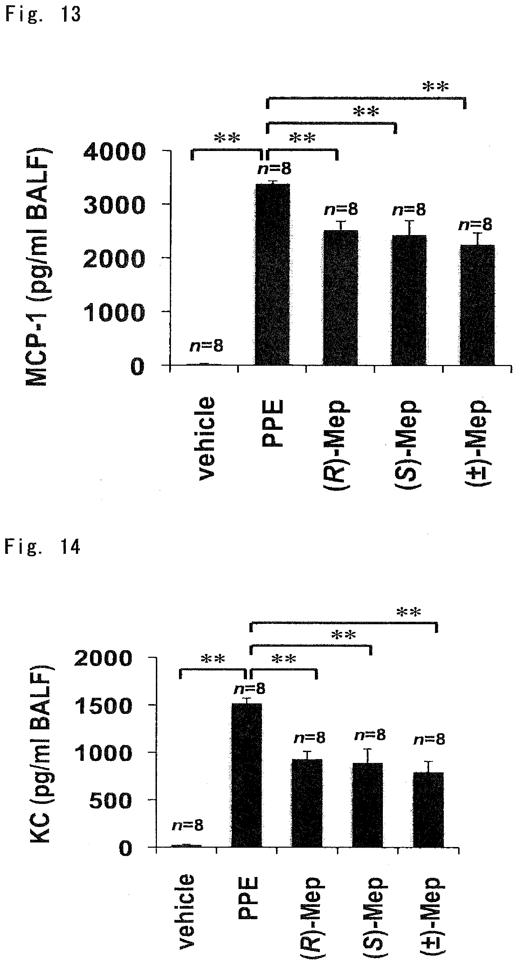

These results were shown in Figs. 11 to 14.

Figs. 11, 12, 13, and 14 show the results of the measurement of TNF-α, MIP-2, MCP-1, and KC, respectively.

As is found from the results shown in the respective figures, each of the racemate, the R-form, and the S-form of mepenzolate bromide was found to have decreased inflammatory factors effectively.

As described above, mepenzolate bromide is known as an anticholinergic drug that has an effect of suppressing movement and contraction in a lower gastrointestinal tract and has been used as a therapeutic drug for irritable bowel in a clinical setting.

Therefore, one example of the side effects due to the anticholinergic action that affect a digestive system is constipation. Mepenzolate bromide also requires careful administration to a patient with arrhythmia since mepenzolate bromide may worsen the symptom by accelerating movement of the heart.

Thus, the level of such side effects caused by different forms of mepenzolate bromide, that is, a racemate, an R-form, and an S-form of mepenzolate bromide was examined.

### Test Example 5: Examination of Fecal Pellet Output (Level of Constipation Occurrence) After Application of Stress

### <Method>

4 to 6 week old ICR mice were put under stress by placing the mice in a 50 mL tube for one hour, the tube ensuring free breathing but restraining free movement, and the fecal pellet output (the level of constipation occurrence) at that time was measured.

Each of a racemate, an R-form, and an S-form of mepenzolate bromide was administered intratracheally to the mice at a dose of 4.7 mg/kg, and one hour after administration, stress was applied to the mice by one-hour exposure to the above-mentioned restrictive stress. The level of constipation occurrence was monitored by measuring the fecal pellet output during the above-mentioned period.

### <Results>

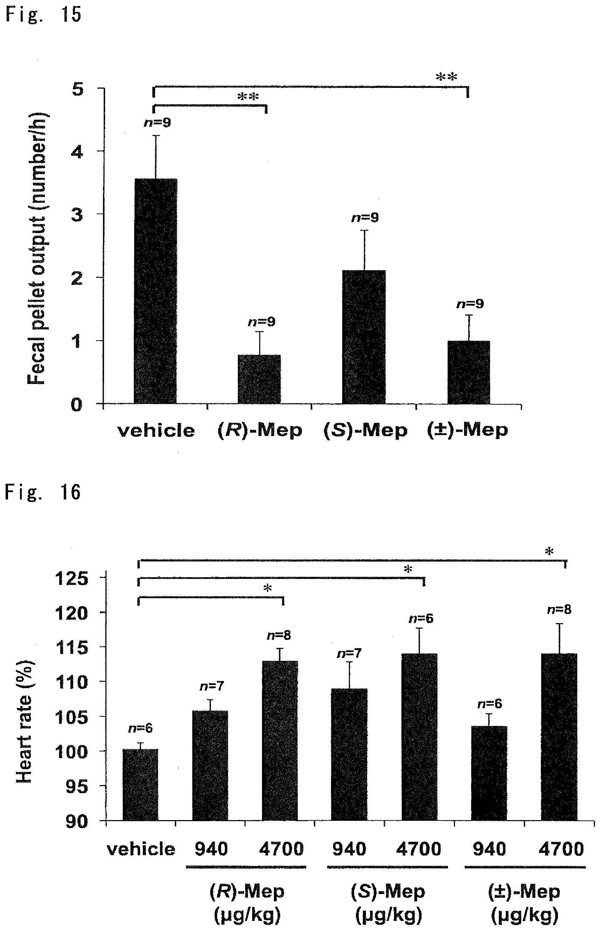

The results were shown in Fig. 15.

As is found from the results shown in the figure, the S-form of mepenzolate bromide led to a low level of constipation occurrence and the R-form of mepenzolate bromide led to the highest level of constipation occurrence.

### Test Example 6: Examination of Effect on Heart Rate

### <Method>

4 to 6 week old ICR mice were anesthetized with chloral hydrate (500 mg/kg) and fitted with a sensor on the femoral region, and the heart rates were measured by the MouseOx system (manufactured by STARR Life Sciences Corp.).

Each of a racemate, an R-form, and an S-form of mepenzolate bromide was administered intratracheally to the mice at a dose of 940 µg/kg and 4700 µg/kg and the heart rates were measured to determine the heart rate exceeding a normal baseline heartbeat caused by administration of mepenzolate bromide.

### <Results>

The results were shown in Fig. 16. The normal heart rate when a vehicle was administered (no administration of mepenzolate bromide) was regarded as 100% and the results were expressed as a percentage relative to the normal heart rate.

As is found from the results shown in the figure, all of the racemate, the R-form, and the S-form of mepenzolate bromide enhanced the heart rate dose-dependently to a similar extent.

The following was concluded from the above-mentioned Test Examples: in terms of an effect of suppressing airway resistance of the R-form and the S-form of mepenzolate bromide, the R-form of mepenzolate bromide tended to have a stronger effect compared to the S-form at low doses (Test Example 3.1), while both the R-form and the S-form had a similar effect at high doses (Test Example 3.2).

In contrast, in terms of an anti-inflammatory effect, no significant difference was observed between the R-form and the S-form of mepenzolate bromide (Test Examples 4.1, 4.2, and 4.3).

Furthermore, when the R-form and the S-form of mepenzolate bromide were compared in terms of a side effect, the S-form suppressed occurrence of constipation more effectively than the R-form.

An agent for ameliorating COPD provided by the present invention ameliorates symptoms of chronic obstructive pulmonary disease by a bronchodilator effect and an anti-inflammatory effect of the optically active form (an R-form and an S-form) of mepenzolate bromide.

Therefore, it was shown that a highly effective agent for ameliorating COPD may be obtained by taking the balance of the strength of both effects (a bronchodilator effect and an anti-inflammatory effect) into consideration and including either of the optically active forms as an active ingredient.

Hereinbelow, exemplary specific formulations of the agent for ameliorating COPD of the present invention are described.

### Formulation Example 1: Inhalation Formulation

A liquid formulation for inhalation is prepared by mixing 1% (w/w) of (R)-mepenzolate bromide, 0.05% (w/w) of benzalkonium chloride, 10% (w/w) of polyethylene glycol, 20% (w/w) of propylene glycol, and the remaining percentage of purified water.

### Formulation Example 2: Inhalation Formulation

A liquid formulation for inhalation is prepared by mixing 1% (w/w) of (S)-mepenzolate bromide, 0.05% (w/w) of benzalkonium chloride, 10% (w/w) of polyethylene glycol, 20% (w/w) of propylene glycol, and the remaining percentage of purified water.

### INDUSTRIAL APPLICABILITY

As described above, the agent for ameliorating COPD provided by the present invention contains an optically active form of mepenzolate bromide, which has already been used in a clinical setting, as an active ingredient. The inventive agent exhibited a remarkable effect of ameliorating COPD by airway administration and inhalation administration, and also exhibited an effect of ameliorating COPD by oral administration and rectal administration. Therefore, the medical contribution thereof is great.

## Claims

1. (3R)-3-[(hydroxy)(diphenyl)acetoxy]-1,1-dimethylpiperidinium bromide (hereinafter referred to as "(R)-mepenzolate bromide") that has a configuration represented by the following formula:

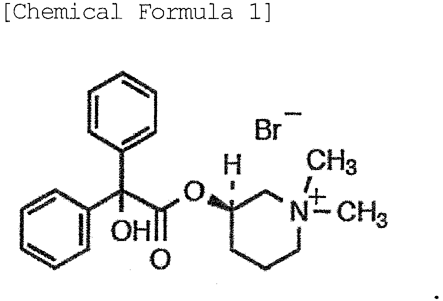

2. (3S)-3-[(hydroxy)(diphenyl)acetoxy]-1,1-dimethylpiperidinium bromide (hereinafter referred to as "(S)-mepenzolate bromide") that has a configuration represented by the following formula:

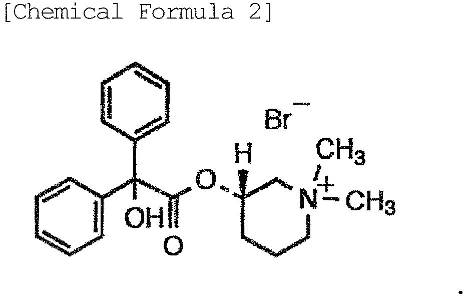

3. An agent for ameliorating chronic obstructive pulmonary disease, comprising the (R)-mepenzolate bromide according to claim 1.

4. An agent for ameliorating chronic obstructive pulmonary disease, comprising the (S)-mepenzolate bromide according to claim 2.

5. The agent for ameliorating chronic obstructive pulmonary disease according to claim 3 or 4, a mode of administration of the agent is airway administration or inhalation administration.

6. A method for producing the (R)- or (S)-mepenzolate bromide according to claim 1 or 2, comprising allowing benzilic acid to react with (R)- or (S)-3-hydroxy-1-methylpiperidin in the presence of a condensing agent and then allowing the obtained product to be treated with methyl bromide.
